# EUROPEAN PATENT SPECIFICATION

(11) **EP 1 163 903 B1**
(45) Date of publication and mention of the grant of the patent: **23.07.2008**
(21) Application number: 01401201.7
(22) Date of filing: 11.05.2001
(51) Int. Cl.: A61K 31/115, A61P 31/00, A61K 9/08

(54) **Methanal injection treatment for animal epidemics**
Methanal - Injektion zur Behandlung epidemischer Tierkrankheiten
Injections de méthanal pour le traitement de maladies animales épidémiques

(30) Priority: 15.05.2000 CN 00113430
(43) Date of publication of application: 19.12.2001
(73) Proprietor: Liu, Zongshan, Wangcheng County, Hunan Province 410204 (CN)
(72) Inventor: Liu, Zongshan, Wangcheng County, Hunan Province 410204 (CN)
(74) Representative: Chaillot, Geneviève

(56) References cited:
- WO-A-88/09655
- WO-A-96/28025
- US-A- 4 833 165
- DATABASE WPI Section Ch, Week 200055 Derwent Publications Ltd., London, GB; Class B05, AN 2000-585658 XP002189589 & RU 2 146 134 C (LASKAVY V N), 10 March 2000 (2000-03-10)
- DATABASE WPI Section Ch, Week 199538 Derwent Publications Ltd., London, GB; Class B05, AN 1995-291235 XP002189575 & RU 2 028 804 C (SARAT RES VETERINARY STATIONIYA), 20 February 1995 (1995-02-20)

## Description

The present invention relates in general to a Methanal Injection that can be used to treat Animal Epidemics.

Animal Epidemics, such as hog plague, fowl plague, tetanus, aftosa, and cynolyssa, are all virus infections with extremely high mortality. Generally, they can be prevented by vaccine inoculation. There are some therapeutics employed to control those diseases, like antisuiseptic (or hog plague) sera. However it is only effective to the initial stages of hog plague, and it is troublesome to prepare, costs a lot. Methanal is a common disinfectant and antiseptic for external use, but no therapeutic applications are reported.

This invention aims to provide a potential methanal injection for animal epidemics, which can be made easily and cheaply, with exact effect and side effects free.

The injection is 0.01 to 4% by weight methanal aqueous solution. Its concentration in use is determined by the selected administration methods - Intravenous injection (I.V.) or Intramuscular injection (I.M.), and also by different animal species, weights, diseases as well as symptoms.

For Intravenous injection, medicine direct entering the vein contributes to a quick onset, so the lower concentration could be selected, usually 0.01 to 0.2% by weight. For Intramuscular injection, the concentration should be higher, usually 0.1 to 4% by weight, depending on the type of animals and diseases. Topical courbature would occur when methanal is applied intramuscularly alone. Adding 0.1 to 2% by weight alcohol, such as glycerine, ethanol, phenmethylol (hydroxymethylbenzene, C₆H₅CH₂OH) or methanol, to methanal solution will act as analgesic. In order to offset the possible adverse effects resulted from formic acid produced by combination between methanal and oxygen of animal bodies, saleratus (or sodium bicarbonate) (tablets or powder) should be taken orally. The saleratus can neutralize the formic acid generated as above, thus escaping the adverse effects. The chemical equations are as follows,

HCOH + O₂ → HCOOH

HCOOH + NaHCO₃ → HCOONa+ H₂O + CO₂

This therapy has been practiced by clinical trials for many years and cured over 1,000 hog plagues, hundreds of fowl plagues, more than 50 aftosas, and many tetanus hogs, tetanus cattle, cynolyssa hogs, pulmo-infections, etc. It is confirmed that methanal injection is indicated in animal epidemics caused by every kind of virus and bacteria. This injection shows actual effect not only to the incipient hog plague, but also to the metaphase. Hogs plague apart, other plagues can be cured by just one shot. Plague hogs' fever comes down after one administration, but may have a recidivation. If it happens, additional hog plague vaccine will be necessary and another methanal injection is needed after 3 to 5 days. All animals after treatments grow normally, and there is no influence on the female hog's genitality.

The following examples are given in order to illustrate the invention and not to limit its scope.

### 1. Fowl plague treated by methanal injection

| **Number of Animals** | **Administration route** | **Concentration of methanal (% by weight)** | **Dose** | **Dose of saleratus** | **Results** |
|---|---|---|---|---|---|
| 150 | I.M. | 0.1∼1% | 5mL | 0.5g/tablet×4 | Cured in 1∼2 days |

### 2. Hog plague treated by methanal injection

| **Number of Animals** | **Administration route** | **Concentration of methanal (% by weight)** | **Dose** | **Dose of saleratus** | **Results** |
|---|---|---|---|---|---|
| Over 1000 | I.M. | 0.1∼2% | 150∼250mL | 0.5g / tablet × 40∼200 tablets | Fever came down after one administration, but had recidivation, additional vaccine was given and another methanal injection was added after 3 to 5 days. All cured. |
| 300 % | I.V. | 0.02∼0.04 | 30∼150mL | 0.5g / tablet × 40∼200 tablets | |

### 3. Other animal epidemics treated by methanal injection

| **Epidemics** | **Number of Animals** | **Administration route** | **Concentration of methanal (% by weight)** | **Dose** | **Results** |
|---|---|---|---|---|---|
| Aftosa Hog | 50 | I.M. | 0.7~4% | 20mL | Cured in 1∼2 days |
| Tetanus | 6 | I.M. | 0.7∼4% | 20mL | Cured in 1∼2 days |
| Cynolyssa | 3 | I.M. | 0.7∼4% | 20mL | Cured in 1∼2 days |
| Pulmoinfection | 2 | I.M. | 0.7∼4% | 20mL | Cured in 1∼2 days |

## Claims

1. Use of an injection aqueous solution for the preparation of a medicament or formulation for the treatment of animal epidemics selected from hog plague, fowl plague, tetanus, aftosa and cynolyssa, wherein the aqueous solution is a 0.01 to 4% by weight methanal aqueous solution.

2. The use according to claim 1, wherein the aqueous solution is a 0.01 to 0.2% by weight methanal aqueous solution for intravenous injection.

3. The use according to claim 1, wherein the aqueous solution is a 0.1 to 4% by weight methanal aqueous solution for intramuscular injection.

4. The use according to any one of claims 1 to 3, wherein the aqueous solution contains 0.1 to 2% by weight alcohol.

## Patentansprüche

1. Verwendung einer wässrigen Injektionslösung zur Herstellung eines Arzneimittels oder einer Formulierung zur Behandlung einer Tierepidemie, ausgewählt aus der Schweinepest, der Vogelgrippe, Tetanus, der Maul- und Klauenseuche und der Hundetollwut, wobei die wässrige Lösung eine wässrige Lösung mit 0,01 bis 4 Gew.-% Methanal ist.

2. Verwendung nach Anspruch 1, wobei die wässrige Lösung eine wässrige Lösung mit 0,01 bis 0,2 Gew.-% Methanal zur intravenösen Injektion ist.

3. Verwendung nach Anspruch 1, wobei die wässrige Lösung eine wässrige Lösung mit 0,1 bis 4 Gew.-% Methanal zur intramuskulären Injektion ist.

4. Verwendung nach einem der Ansprüche 1 bis 3, wobei die wässrige Lösung 0,1 bis 2 Gew.-% Alkohol enthält.

## Revendications

1. Utilisation d'une solution aqueuse pour injection pour la préparation d'un médicament ou d'une formulation pour le traitement d'une épidémie animale choisie parmi la peste du porc, la peste aviaire, le tétanos, la fièvre aphteuse et la rage du chien, dans laquelle la solution aqueuse est une solution aqueuse à 0,01 à 4 % en poids de méthanal.

2. Utilisation selon la revendication 1, dans laquelle la solution aqueuse est une solution aqueuse à 0,01 à 0,2% en poids de méthanal pour injection intraveineuse.

3. Utilisation selon la revendication 1, dans laquelle la solution aqueuse est une solution aqueuse à 0,1 à 4% en poids de méthanal pour injection intramusculaire.

4. Utilisation selon l'une quelconque des revendications 1 à 3, dans laquelle la solution aqueuse contient 0,1 à 2% en poids d'alcool.
